# EUROPEAN PATENT SPECIFICATION

(11) **EP 4 563 183 B1**
(45) Date of publication and mention of the grant of the patent: **21.10.2026**
(21) Application number: 23897367.1
(22) Date of filing: 01.11.2023
(51) Int. Cl.: A61M 25/10

(54) **BALLOON CATHETER AND METHOD OF MANUFACTURING THE SAME**
BALLONKATHETER UND VERFAHREN ZUR HERSTELLUNG DAVON
CATHÉTER À BALLONNET ET SON PROCÉDÉ DE FABRICATION

(30) Priority: 30.11.2022 JP 2022192304
(43) Date of publication of application: 04.06.2025
(73) Proprietor: Nipro Vascular Corporation, Nagoya-shi, Aichi 460-0008 (JP)
(72) Inventor: OKAMOTO, Mitsumasa, Seto-shi, Aichi 489-0976 (JP)
(74) Representative: Prüfer & Partner mbB Patentanwälte · Rechtsanwälte
(86) International application number: PCT/JP2023/039484
(87) International publication number: WO 2024/116711

(56) References cited:
- EP-A1- 3 795 200
- WO-A1-2020/012851
- WO-A1-2020/255923
- JP-A- 2002 539 889
- US-A1- 2003 004 535
- US-A1- 2006 182 873
- US-A1- 2007 267 780
- US-A1- 2015 105 723
- US-A1- 2019 216 297
- US-A1- 2020 276 417

## Description

### Cross-Reference to Related Application

The present application is based on Japanese Patent Application No. 2022-192304 filed on November 30, 2022.

### Technical Field

The present disclosure relates to a balloon catheter and a method of producing a balloon catheter.

### Background Art

A balloon catheter has been used in percutaneous transluminal angioplasty (PTA), percutaneous transluminal coronary angioplasty (PTCA) and other surgeries. The balloon catheter includes an inflatable and deflatable balloon at a tip-end portion of the balloon catheter (see PLT1 for example). The balloon of the balloon catheter may be deflated and inserted into a section of the blood vessel that is narrowed or obstructed by a lesion and then inflated to dilate the section.

US 2003/004535 A1 discloses a medical balloon having at least one position marking thereon at the middle of the balloon, wherein the marked surface may consist of a roughened area of the balloon.

US 2015/105723 A1 discloses a medical balloon formed by a parison, wherein the balloon may be formed having an etched or textured outer surface layer of the balloon wall.

US 2006/182873 A1 describes a method including providing a medical balloon comprising a first cone portion and a body portion and removing material from an outer surface of the body portion to form a first regions that is recessed relative to a second region.

EP 3 795 200 A1 discloses a balloon catheter comprising a balloon formed from a film part that has a prescribed thickness, the film part being provided with linear projections.

### Citation List

### Patent Literature

PLT1: International Publication WO2020/255923.

### Summary of Invention

The aforementioned objectives are achieved according to the invention by means of a balloon catheter according to claim 1 and equally by means of a method of producing such a balloon catheter according to claim 5. Preferred embodiments are defined in the dependent claims.

### Technical Problem

When the balloon is inflated to dilate a section with a lesion in the body, the lesion may push the balloon and thus the balloon may slip in the axial direction. If that happens, the balloon may not be able to properly dilate the section with the lesion.

To reduce the slipping of the balloon during the inflation of the balloon, surface roughness of the outer surface of the straight tube portion of the balloon may be increased. For example, the surface roughness of the entire outer surface of the balloon with respect to the axial direction of the balloon may be increased. However, such a configuration may increase a sliding resistance of the balloon during the insertion of the balloon into a tube such as a blood vessel and may decrease ease in passing of the balloon through the tube.

The present disclosure has been made in view of the above circumstances. A main objective of the present disclosure is to provide a balloon catheter that includes a balloon that is less likely to slip during inflation of the balloon without a reduction in ease in passing of the balloon through a tube and to provide a method of producing the balloon catheter.

### Solution to Problem

To solve the problem described above, a first aspect of the present disclosure provides a balloon catheter that includes a balloon that is inflatable and deflatable. The balloon includes an outer surface that includes a first region and a second region that is at a part of the balloon in an axial direction of the balloon and has surface roughness greater than surface roughness of the first region.

In the first aspect, the outer surface of the balloon includes the first region and the second region that has the surface roughness greater than the surface roughness of the first region. According to the configuration, the second region contacts a target section to be dilated such as a section with a lesion during the inflation of the balloon and thus the balloon is less likely to slip. Further, the second region is present only at the part of the balloon with respect to the axial direction of the balloon and other parts of the balloon includes the first region that has the less surface roughness. In comparison to a configuration in which the second region is present for an entire area of the balloon with respect to the axial direction of the balloon, the sliding resistance of the balloon during insertion of the balloon into a tube such as a blood vessel is reduced. According to the configuration, the slipping of the balloon during the inflation of the balloon is reduced without a reduction in ease in passing of the balloon through the tube.

In a second aspect of the present disclosure, the balloon in the first aspect includes a straight tube portion and two tapered portions. The straight tube portion has a diameter that is to be maximum when the balloon is inflated. The tapered portions sandwich the straight tube portion with respect to the axial direction. The tapered portions decrease in diameter toward the straight portion. The second region is formed in an outer surface of one of the tapered portions.

During the dilation of the section with the lesion using the tapered portions of the balloon, the tapered portions contact the lesion at an angle and thus the tapered portions may be pushed back by the lesion at an angle, which may cause slipping of the balloon in the axial direction. In the second aspect, the outer surface of one of the tapered portions includes the second region that has the greater surface roughness. With the second region that contacts the lesion during the inflation of the balloon, one of the tapered portions are less likely to slip in the axial direction and thus the balloon is less likely to slip in the axial direction.

In a third aspect of the present disclosure, the second region in the second aspect is formed in the outer surface of one of the tapered portions on a tip-end side.

If the lesion in the tube such as a blood vessel is large, a lumen of the section with the lesion is narrowed. Therefore, the straight tube portion of the balloon may not be inserted into the lumen of the section with the lesion. In such a case, the tapered portion on the tip-end side with a relatively small diameter may be inserted into the section with the lesion and the balloon may be inflated while the tapered portion on the tip-end side is inserted in the section with the lesion such that the section with the lesion may be dilated by the tapered portion on the tip-end side.

When the section with the lesion is dilated as described above, the tapered portion on the tip-end side may be pushed toward the base-end side (the proximal side) by the lesion and the tapered portion on the tip-end side may slip toward the base-end side. The third aspect is conceived in view of such circumstances and the second region is formed in the outer surface of the tapered portion on the tip-end side. According to the configuration, the tapered portion on the tip-end side is less likely to slip toward the base-end side and thus the balloon is less likely to slip toward the base-end side during the dilation of the section with the lesion.

In a fourth aspect of the present disclosure, the balloon catheter in the third aspect includes a catheter tube that includes a tip-end portion at which the balloon is disposed. The balloon includes a tip-end leg portion that is on an opposite side of the tapered portion on the tip-end side from the straight tube portion. The tip-end leg portion is joined to the catheter tube. The second region is formed in an outer surface of the tip-end leg portion.

If the lumen of the section with the lesion is narrowed, the tapered portion on the tip-end side and the tip-end leg portion may be inserted into the lumen of the section with the lesion and then the balloon may be inflated. In the fourth aspect, the second region is formed in the outer surface of the tip-end leg portion in addition to the tapered portion on the tip-end side. According to the configuration, the balloon is further less likely to slip toward the base-end side during the inflation of the balloon.

In a fifth aspect of the present disclosure, the first region of the balloon catheter in any one of the second to the fourth aspect is formed in an outer surface of the straight tube portion.

Because the straight tube portion has the maximum diameter among the portions of the balloon, the straight tube portion is more likely to slide on a tube wall during the insertion of the balloon into the tube. In the fifth aspect, the outer surface of the straight tube portion includes the first region that has the less surface roughness and thus the sliding resistance of the balloon during the insertion of the balloon into the tube is properly reduced. According to the configuration, the ease in passing of the balloon is properly reduced.

In a sixth aspect of the present disclosure, the second region of one of the tapered portions has surface roughness that decreases toward the straight tube portion.

A section of the tapered portion adjacent to the straight tube portion has a relatively large diameter. Therefore, the section of the tapered portion adjacent to the straight tube portion is more likely to slide on the tube wall during the insertion of the balloon into the tube. The sixth aspect is conceived in view of such circumstances and the surface roughness of the second region of the tapered portion decreases toward the straight tube portion. According to the configuration, the sliding resistance of the section of the tapered portion adjacent to the straight tube portion during the insertion of the balloon into the tube is reduced. Therefore, the sliding resistance of the balloon during the insertion of the balloon into the tube is further reduced.

In a seventh aspect of the present disclosure, the balloon catheter in the second aspect includes a catheter tube that includes a tip-end portion at which the balloon is disposed. The balloon includes a leg portion that is on an opposite side of one of the tapered portions from the straight tube portion. The leg portion is joined to the catheter tube. The second region is formed in an outer surface of the leg portion.

If the lumen of the section with the lesion is narrowed, the tapered portion and the leg portion may be inserted into the lumen of the section with the lesion. In the seventh aspect, the second region is formed in the outer surface of the leg portion in addition to the outer surface of the tapered portion. According to the configuration, the balloon is further less likely to slip during the inflation of the balloon.

In an eighth aspect of the present disclosure, the second region of the balloon catheter in any one of the first to the fourth aspect is present for an entire area of the balloon with respect to a circumferential direction of the balloon.

In the eighth aspect, the second region is present for the entire area of the balloon with respect to the circumferential direction of the balloon and thus the second region properly contacts a target section to be dilated such as the section with the lesion during the inflation of the balloon. According to the configuration, the slipping of the balloon during the inflation of the balloon is properly reduced.

In a ninth aspect of the present disclosure, the balloon catheter in the second aspect includes an internal protrusion that protrudes from an inner surface of the one of the tapered portions and extends in the axial direction along the inner surface. The tapered portion on which the internal protrusion is disposed includes an outer surface that includes an overlapping region that overlaps the internal protrusion with respect to the thickness direction of the tapered portion. The second region is in an area of the outer surface of the tapered portion on which the internal protrusion is disposed. The area includes the overlapping region.

In the ninth aspect, the internal protrusion protrudes from the inner surface of the tapered portion of the balloon and extends in the axial direction. Because a section of the tapered portion on which the internal protrusion is disposed is increased in strength, the section of the tapered portion properly contacts the lesion during the dilation of the section with the lesion using the tapered portion of the balloon that is inflated. According to the configuration, the tapered portion is less likely to be pushed back by the lesion during the dilation of the section with the lesion using the tapered portion.

Further, the overlapping region of the outer surface of the tapered portion overlapping the internal protrusion with respect to the thickness direction of the tapered portion properly contacts the lesion during the dilation of the section with the lesion using the tapered portion. In the ninth aspect, the second region is present in the area of the outer surface of the tapered portion including the overlapping region. According to the configuration, the second region in the overlapping region properly contacts the lesion. Therefore, the slipping of the balloon is properly reduced.

In a tenth aspect of the present disclosure, the second region of the balloon catheter in the ninth aspect includes a predetermined area in the overlapping region. The predetermined area has surface roughness greater than surface roughness of any other area of the second region.

In the tenth aspect, the surface roughness of the predetermined area of the second region in the overlapping region is greater than the surface roughness of any other area of the second region. Because the surface roughness in the overlapping region that properly contacts the lesion is greater, the advantageous effect in reduction of the slipping of the balloon is enhanced.

In an eleventh aspect of the present disclosure, the balloon catheter in the ninth aspect or the tenth aspect includes an external protrusion that protrudes from an outer surface of the straight tube portion and extends in the axial direction. The internal protrusion and the external protrusion are disposed at the same position with respect to a circumferential direction of the balloon.

In the eleventh aspect, the external protrusion is disposed on the straight tube portion of the balloon. Therefore, the external protrusion creates incisions in the lesion during the inflation of the balloon and the section with the lesion can be easily dilated using the incisions as a trigger of the dilation. Further, the internal protrusion on the tapered portion and the external protrusion on the straight tube portion are disposed at the same position with respect to the circumferential direction of the balloon. The internal protrusion and the external protrusion having greater strength are disposed in a row with respect to the axial direction and thus the tapered portion is further less likely to be pushed back by the lesion during the dilation of the section with the lesion using the tapered portion.

A twelfth aspect of the present disclosure provides a method of producing the balloon catheter in the fifth aspect. The method includes producing the balloon using a mold that includes an internal space for forming the balloon. The internal space includes a first space section for forming the straight tube portion and a second space section for forming one of the tapered portions. The mold includes an inner wall surface that defines the internal space. The inner wall surface includes a first surface section that defines the first space section and a second surface section that defines the second space section. The second surface section has surface roughness greater than surface roughness of the first surface section. The method includes a placing process of placing a parison in the internal space and an expanding process of expanding the parison in the internal space. The parison is in a tubular shape and a base material of the balloon. The expanding process includes forming the first region in the outer surface of the straight tube portion by pressing an outer periphery of the parison, which is expanded, against the first surface section, and forming the second region in the outer surface of the tapered portion by pressing the outer periphery of the parison, which is expanded, against the second surface section.

In the twelfth aspect, the first region is formed in the outer surface of the straight tube portion and the second region is formed in the outer surface of the tapered portion during the expansion of the parison in the internal space of the mold. A downstream process such as scraping of the outer surface of the tapered portion to form the second region is not required after the balloon is formed by expanding the parison. Therefore, the balloon catheter in the fifth aspect is relatively easily produced.

In a thirteen aspect of the present disclosure, the method in the twelfth aspect is for producing the balloon catheter that includes an external protrusion that protrudes from the outer surface of the straight tube portion and extends in the axial direction and an internal protrusion that protrudes from the inner surface of the tapered portion and extends in the axial direction along the inner surface. The parison includes a protrusion that protrudes from an outer periphery of the parison and extends in a longitudinal direction of the parison. The first surface section includes a groove that extends in a longitudinal direction of the internal space. The second surface section does not include the groove. The expanding process includes: forming the external protrusion by inserting the protrusion of the parison, which is expanded, into the groove; forming the internal protrusion by pressing the protrusion of the parison, which is expanded, against the second surface section until the protrusion is squashed and a portion of the parison along the protrusion protrudes inward; and forming the second region in a surface that is in close contact with the second surface section during squashing of the protrusion.

In the thirteenth aspect, the parison that includes the protrusion is expanded in the internal space of the mold. As a result, the external protrusion and the internal protrusion are formed on the straight tube portion and the tapered portion of the balloon, respectively. Specifically, the protrusion of the parison is pressed against the second surface section of the mold until the protrusion is squashed. As a result, the portion of the parison protrudes inward and the internal protrusion is formed. During the squashing of the protrusion, the second region is formed in the surface that is in close contact with the second surface section. The second region having the greater surface roughness is formed in the overlapping region that overlaps the internal protrusion in the thickness direction of the tapered portion. According to the configuration, the balloon catheter in the ninth aspect is relatively easily produced.

In a fourteen aspect of the present disclosure, the second surface section that defines the second space section in the thirteenth aspect includes a protruded surface that is a part of the second surface section with respect to a circumferential direction of the second surface section that is protruded inward. The expanding process includes pressing the protrusion against the protruded surface until the protrusion is squashed.

In the fourteenth aspect, the protrusion of the parison is pressed against the protruded surface that protrudes inward and thus the protrusion is strongly pressed against the second surface section (the protruded surface). According to the configuration, the surface roughness of the second region in the overlapping region is further increased.

### Brief Description of Drawings

The above-described object and any other object, features, and advantages of the present disclosure will be further clarified by the following detailed description made with reference to the attached drawings.
[FIG. 1] A schematic overall side view illustrating a configuration of a balloon catheter according to a first embodiment.
[FIG. 2] (a) A side view illustrating a configuration of a balloon and therearound, and (b) a side view of the balloon and therearound with a lengthwise cross-sectional view of the balloon and an outer tube.
[FIG. 3] A cross-sectional view illustrating a configuration of a mold with a parison placed inside the mold.
[FIG. 4] An explanatory view illustrating a method of using the balloon catheter, which does not form part of the invention
[FIG. 5] A side view illustrating a configuration of a balloon and therearound according to a second embodiment.
[FIG. 6] (a) A side view illustrating a configuration of a balloon and therearound according to a third embodiment, (b) a cross-sectional view cut along line A-A in FIG. 6(a), (c) a cross-sectional view cut along line B-B in FIG. 6(a), and (d) a cross-sectional view cut along line C-C in FIG. 6(a).
[FIG. 7] A side view of the balloon and therearound with a lengthwise cross-sectional view of the balloon and an outer tube.
[FIG. 8] A perspective view of a parison.
[FIG. 9] (a) A cross-sectional view illustrating a configuration of a mold, (b) a cross-sectional view cut along line D-D in FIG. 9(a), (c) a cross-sectional view cut along line E-E in FIG. 9(a), and (d) a cross-sectional view cut along line F-F in FIG. 9(a).
[FIG. 10] cross-sectional views illustrating the parison that is inflated in an internal space of the mold, (a) a cross-sectional view cut along line D-D in FIG. 9(a), (b) a cross-sectional view cut along line E-E in FIG. 9(a), and (c) a cross-sectional view cut along line F-F in FIG. 9(a).

### Description of Embodiments

### First Embodiment

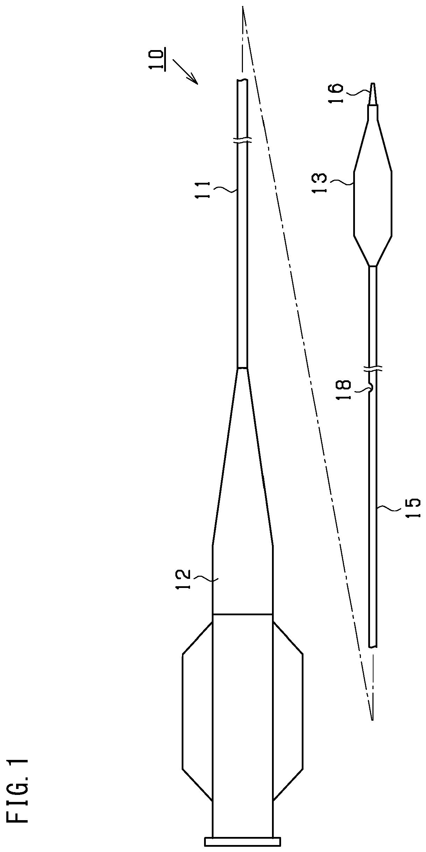

An embodiment of the present disclosure will be described with reference to the drawings. FIG. 1 is a schematic overall side view illustrating a configuration of a balloon catheter.

As illustrated in FIG. 1, a balloon catheter 10 includes a catheter tube 11, a hub 12, and a balloon 13. The hub 12 is mounted to a base-end portion (a proximal portion) of the catheter tube 11. The balloon 13 is mounted to a tip-end portion (a distal portion) of the catheter tube 11.

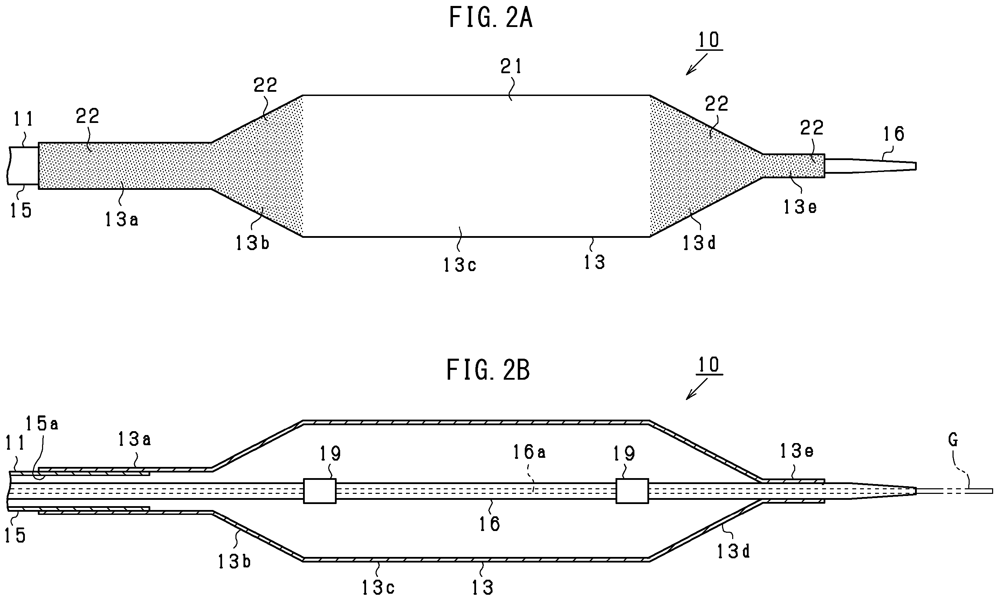

The catheter tube 11 includes an outer tube 15 and an inner tube 16 that is inserted in the outer tube 15. The outer tube 15 is made of a resin material and formed in a tubular shape. The outer tube 15 includes a fluid lumen 15a inside (see FIG. 2(b)). The fluid lumen 15a extends through the outer tube 15 in an axial direction of the outer tube 15 for an entire length. A base-end portion of the outer tube 15 is joined to the hub 12, and the tip-end portion of the outer tube 15 is joined to the balloon 13. The fluid lumen 15a of the outer tube 15 is communicated with an internal space of the hub 12 and an internal space of the balloon 13. For inflation and deflation of the balloon 13, a compressed fluid is passed through the fluid lumen 15a of the outer tube 15.

The outer tube 15 may be composed of multiple tubes that are disposed in a row with respect to the axial direction and joined to one another. One of the multiple tubes on the base-end side may be made of a metal material and another one of the multiple tubes on the tip-end side may be made of a resin material.

The inner tube 16 is made of a resin material and formed in a tubular shape. The inner tube 16 includes a guidewire lumen 16a inside (see FIG. 2(b)). A base-end portion of the inner tube 16 is joined to a middle portion of the outer tube 15 with respect to the axial direction of the outer tube 15. A portion of the inner tube 16 on the tip-end side extends farther than a tip of the outer tube 15 and inserted in the internal space of the balloon 13. A tip-end portion of the inner tube 16 is joined to a tip-end portion of the balloon 13.

The guidewire lumen 16a of the inner tube 16 is provided for passing a guidewire G. A base-end opening of the guidewire lumen 16a is located in the middle of the balloon catheter 10 with respect to the axial direction of the balloon catheter 10. That is, the balloon catheter 10 is classified as an RX-type catheter. A base-end opening 18 of the guidewire lumen 16a may be located at a base-end portion of the balloon catheter 10. This type of the balloon catheter 10 may be classified as an over-the-wire-type catheter.

A configuration of the balloon 13 and therearound will be described with reference to FIGS. 2(a) and 2(b). FIG. 2(a) is a side view illustrating the configuration of the balloon 13 and therearound. FIG. 2(b) is a side view illustrating a configuration of the balloon 13 and therearound with a lengthwise cross-sectional view of the balloon 13 and the outer tube 15. FIGS. 2(a) and 2(b) illustrate the balloon 13 that is inflated.

The balloon 13 is made of a thermoplastic resin material such as polyamide elastomer. As illustrated in FIGS. 2(a) and 2(b), the balloon 13 is formed in a cylindrical shape (a tubular shape) with a circular cross section as a whole. Specifically, the balloon 13 includes a base-end leg portion 13a, a base-end tapered portion 13b, a straight tube portion 13c, a tip-end tapered portion 13d, and a tip-end leg portion 13e in this sequence from the base-end side toward the tip-end side.

The base-end leg portion 13a is joined to the tip-end portion of the outer tube 15. The base-end tapered portion 13b increases in diameter from the tip-end of the base-end leg portion 13b toward the tip-end side. That is, the base-end tapered portion 13a is in a tapered shape. The straight tube portion 13c extends from the tip-end of the base-end tapered portion 13b toward the tip-end side with a constant diameter. That is, the straight tube portion 13c is in a cylindrical shape. The straight tube portion 13c has a diameter that is to be maximum when the balloon 13 is inflated. The tip-end tapered portion 13d decreases in diameter from the tip-end of the straight tube portion toward the tip-end side. That is, the tip-end tapered portion 13d is in a tapered shape. The tip-end leg portion 13e is joined to the tip-end portion of the inner tube 16.

When the compressed fluid is supplied to the internal space of the balloon 13 via the fluid lumen 15a of the outer tube 15, the balloon 13 inflates. When the negative pressure is applied to the fluid lumen 15a of the outer tube 15 and the compressed fluid is discharged from the internal space of the balloon 13, the balloon 13 deflates. Although not illustrated in the drawing, the balloon 13 that is deflated includes multiple wings that appear when the balloon 13 is deflated. The wings are disposed at predetermined intervals in the circumferential direction of the balloon 13. When the balloon 13 is deflated, the wings are folded along the outer periphery of the balloon 13 and wound around the inner tube 16.

Two contrast rings 19 are attached to the inner tube 16 inside the balloon 13. The contrast rings 19 are for improving visibility of the balloon 13 in x-ray images to easily position the balloon 13 to a target section for treatment.

The outer surface of the balloon 13 of the balloon catheter 10 includes multiple regions that have different degrees of surface roughness. The regions will be described.

As illustrated in FIG. 2(a), the outer surface of the balloon 13 includes a first region 21 and second regions 22. The surface roughness of the first region 21 is less than the surface roughness of the second regions 22. The surface roughness of the second regions 22 is greater than the surface roughness of the first region 21. The first region 21 and the second regions 22 are disposed in a row with respect to the axial direction of the balloon 13. In FIG. 2(a), the second regions 22 are indicated with dot-hatching patterns.

The first region 21 is formed in the outer surface of the straight tube portion 13c of the balloon 13. Specifically, the first region 21 is present for an entire area of the outer surface of the straight tube portion 13c. The surface roughness of the first region 21 is constant for an entire area of the first region 21.

The second regions 22 are formed in the outer surfaces of the base-end leg portion 13a, the base-end tapered portion 13b, the tip-end tapered portion 13d, the tip-end leg portion 13e of the balloon 13. Specifically, the second regions 22 are present for entire areas of the portions 13a, 13b, 13d and 13e of the balloon 13. That is, the second regions 22 are present for the entire areas of the outer surface of the balloon 13 except for the area in which the first region 21 is present (the outer surface of the straight tube portion 13c). The second regions 22 of portions 13a, 13b, 13d and 13e have the same surface roughness. The surface roughness of each of the second regions 22 of the portions 13a, 13b, 13d and 13e is constant for the entire area.

The degrees of the surface roughness of the second regions 22 of the portions 13a, 13b, 13d and 13e can be different from one another. For example, the surface roughness of the second region 22 of each tapered portion 13b, 13d may be greater than the surface roughness of the second region 22 of each leg portion 13a, 13e. Alternatively, the surface roughness of the second region 22 of each tapered portion 13b, 13d may be less than the surface roughness of the second region 22 of each leg portion 13a, 13e.

In this disclosure, the expression "surface roughness" means calculated mean roughness Ra defined in the Japanese industrial standards (JIS) B0601:2001. The calculated mean roughness Ra is measured according to JIS B0601:2001 using instrument specified in JIS B0651:2001.

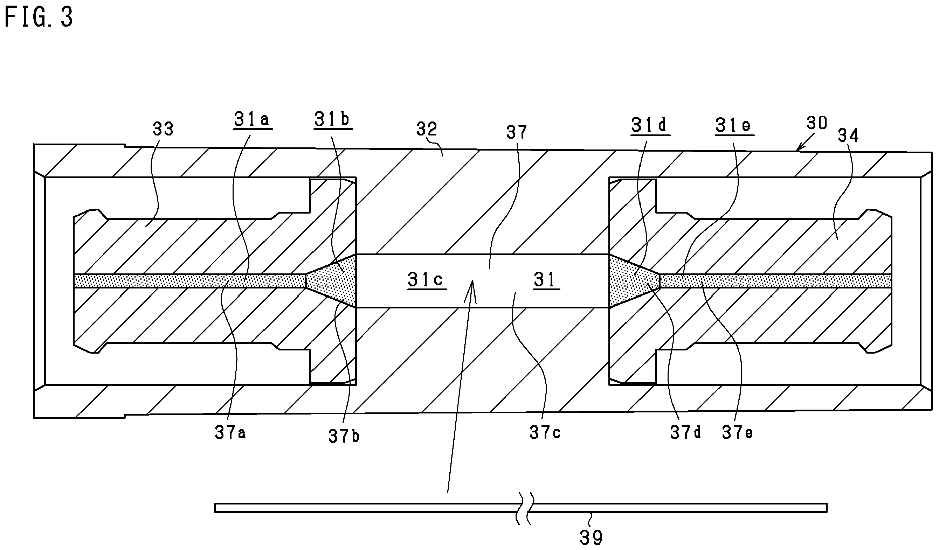

A method of producing the balloon 13 will be described. The balloon 13 is produced using the mold 30. First, a configuration of the mold 30 will be described with reference to FIG. 3. FIG. 3 is a cross-sectional view illustrating the configuration of the mold 30. FIG. 3 also illustrates a parison 39 that is placed inside the mold 30.

As illustrated in FIG. 3, the mold 30 that is formed in a rectangular shape includes an internal space 31 for forming the balloon 13. The internal space 31 is an elongated space that extends in the longitudinal direction of the mold 30 and has a shape corresponds to the shape of the balloon 13. The internal space 31 has a cross section (a cross section along a direction perpendicular to the longitudinal direction of the internal space 31) in a shape that is circular for an entire length of the internal space 31 in the longitudinal direction.

The internal space 31 includes a space section 31a for forming the base-end leg portion 13a, a space section 31b (corresponding to a second space section) for forming the base-end tapered portion 13b, a space section 31c for forming the straight tube portion 13c (corresponding to a first space section), a space section 31d (corresponding to a second space section) for forming the tip-end tapered portion 13d, and a space section 31e for forming the tip-end leg portion 13e. Shapes of the space sections 31a to 31e correspond to shapes of the portions 13a to 13e of the balloon 13, respectively.

The mold 30 includes multiple components that are fitted together. The multiple components include mold components 32 to 34 that define the internal space 31. The mold component 32 includes the space section 31c for forming the straight tube portion 13c. The mold component 33 includes the space section 31b for forming the base-end tapered portion 13b and the space section 31a for forming the base-end leg portion 13a. The mold component 34 includes the space section 31d for forming the tip-end tapered portion 13d and the space section 31e for forming the tip-end leg portion 13e. The configuration of the mold 30 is not limited to the above configuration including multiple mold components. The mold 30 may include a single mold component.

The mold 30 includes an inner wall surface 37 that defines the internal space 31. The inner wall surface 37 includes an inner wall surface 37a that defines the space section 31a for the base-end leg portion 13a, an inner wall surface 37b that defines the space section 31b for the base-end tapered portion 13b (corresponding to a second surface section), an inner wall surface 37c that defines the space section 31c for the straight tube portion 13c (corresponding to a first surface section), an inner wall surface 37d that defines the space section 31d for the tip-end tapered portion 13d, and an inner wall surface 37e that defines the space section 31e for the tip-end leg portion 13e. The surface roughness of the inner wall surface 37c is less than the surface roughness of other inner wall surfaces 37a, 37b, 37d and 37e. The surface roughness of each of the inner wall surfaces 37a, 37b, 37d and 37e is greater than the surface roughness of the inner wall surface 37c. The inner wall surfaces 37a, 37b, 37d and 37e have the same surface roughness. In FIG. 3, the inner wall surfaces 37a, 37b, 37d and 37e are indicated with dot-hatching patterns.

A method of producing the balloon 13 using the mold 30 will be described. To produce the balloon 13, the mold 30 and the parison 39 that is a base material of the balloon 13 are prepared in a preparing process. The parison 39 is made of a resin material and formed in a cylindrical shape. The parison 39 may be formed by extrusion molding.

A placing process is performed to place the parison 39 in the internal space 31 of the mold 30. In the placing process, the parison 39 is placed in the internal space 31 with the longitudinal direction of the parison 39 aligned with the longitudinal direction of the internal space 31.

An expanding process is performed to heat and expand the parison 39 in the internal space 31 of the mold 30. In the expanding process, the parison 39 is expanded by introducing a fluid such as nitrogen into the inside of the parison 39. Through the expanding process, an expanded parison that includes featured portions of the balloon 13 is formed. That is, the expanded parison that includes the portions 13a to 13e of the balloon 13 is formed. Alternatively, a stretching process may be performed prior to the expanding process to stretch the parison 39 in the longitudinal direction. The expanding process may be a blow-molding process in which blow molding is performed.

In the expanding process, the first region 21 is formed in the outer surface of the straight tube portion 13c by pressing the outer periphery of the parison 39, which is expanded, against the inner wall surfaces 37c of the mold 30 (the mold component 32). The second regions 22 are formed in the outer surfaces of the base-end tapered portion 13b and the base-end leg portion 13a by pressing the outer periphery of the parison 39, which is expanded, against the inner wall surfaces 37a and 37b of the mold 30 (the mold component 33), respectively. The second regions 22 are formed in the outer surfaces of the tip-end tapered portion 13d and the tip-end leg portion 13e by pressing the outer periphery of the parison 39, which is expanded, against the inner wall surfaces 37d and 37e of the mold 30 (the mold component 34), respectively.

After the expanding process, the cutting process is performed to cut off extra end portions of the expanded parison. This completes the formation of the balloon 13 and the production of the balloon 13 is complete.

Downstream processes including a bonding process for bonding the balloon 13 to the catheter tube 11 and a connecting process for connecting the catheter tube 11 to the hub 12 are performed. At the completion of the downstream processes, a series of production steps is complete.

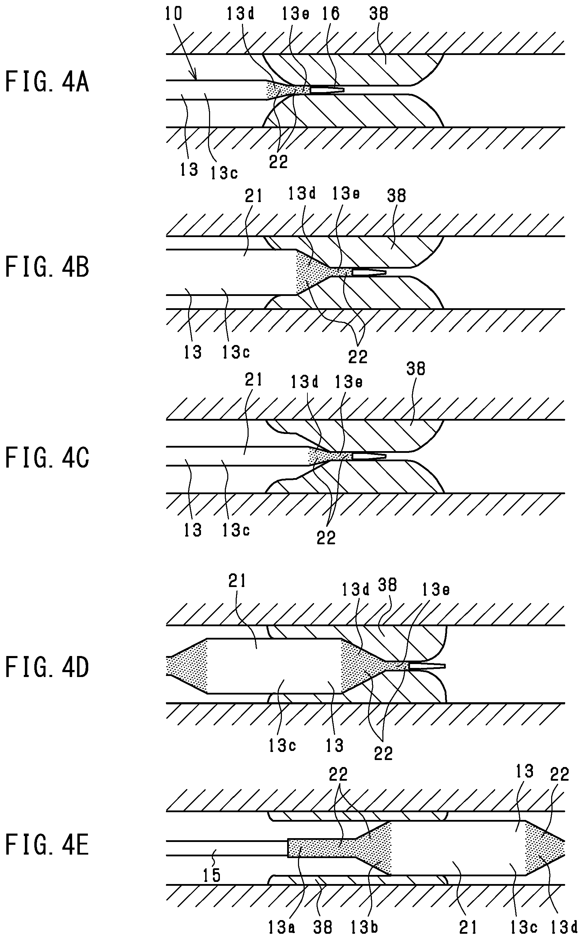

A method of using the balloon catheter 10 will be described, which does not form part of the invention. A procedure of dilating a section of a blood vessel with a lesion using the balloon catheter 10 will be described. FIG. 4 is an explanatory view illustrating the non-claimed method.

A guiding catheter is inserted into a sheath introducer that is in the blood vessel and then a tip opening of the guiding catheter is inserted into a coronary artery inlet. The guidewire G is then inserted into the guiding catheter and the inserted guidewire G is inserted from the coronary artery inlet to a distal section via the section with the lesion.

Then, the balloon catheter 10 is inserted into the guiding catheter along the guidewire G. After the insertion, pushing force and pulling force are applied until the balloon 13 is placed in the section with the lesion 38. During the insertion, the balloon 13 remains deflated.

In the example in FIG. 4(a), the lesion 38 in the blood vessel is relatively large. That is, a lumen of a section with the lesion 38 is significantly narrowed by the lesion 38. Therefore, the balloon 13 may not be properly inserted into the lumen with the lesion 38. For example, the straight tube portion 13c may not be inserted into the lumen of the section with the lesion 38. In such a case, the tip-end tapered portion 13d and the tip-end leg portion 13e of the balloon 13 are inserted into the lumen of the section with the lesion 38 as illustrated in FIG. 4(a) and then the balloon 13 is inflated. As illustrated in FIG. 4(b), the section with the lesion 38 is dilated by the tip-end tapered portion 13d that is inflated.

During the dilation of the section with the lesion 38 using the tip-end tapered portion 13d, the tip-end tapered portion 13d contacts the lesion 38 at an angle and thus the lesion 38 may push back the tip-end tapered portions 13d at an angle, which may cause slipping of the balloon 13 toward the base-end side (the proximal side). In this embodiment, the outer surface of the tip-end tapered portion 13d of the balloon 13 includes the second region 22 that has the greater surface roughness. With the second region 22 in contact with the lesion 38, the tip-end tapered portion 13d is less likely to slip toward the base-end side and thus the balloon 13 is less likely to slip toward the base-end side.

Because the second region 22 is also formed in the outer periphery of the tip-end leg portion 13e, the second region 22 of the tip-end leg portion 13e inserted in the section with the lesion 38 contacts the lesion 38. According to the configuration, the balloon 13 is further less likely to slip toward the base-end side during the dilation of the section with the lesion 38 using the tip-end tapered portion 13d.

After the dilation of the section with the lesion 38 using the tip-end tapered portion 13d is complete, the balloon 13 is deflated as illustrated in FIG. 4(c). Then, the deflated balloon 13 is further moved toward the tip-end side (the distal side) until the tip-end tapered portion 13d and the tip-end leg portion 13e of the balloon 13 reach a section with the lesion 38 in which the dilation is not yet performed. The straight tube portion 13c of the balloon 13 is placed in a cone-shaped dilated section of the section with the lesion 38, which is already dilated by the tip-end tapered portion 13d. The balloon 13 with the straight tube portion 13c that is placed in the cone-shaped dilated section is inflated. As a result, a non-dilated section of the section with the lesion 38 is dilated by the tip-end tapered portion 13d (see FIG. 4(d)) and the cone-shaped dilated section of the section with the lesion 38 is further dilated by the straight tube portion 13c.

Through the steps described above, the non-dilated section with the lesion 38 is gradually dilated from a proximal side to a distal side. When all the steps are performed, the entire section with the lesion 38 is dilated by the balloon 13.

After the dilation of the section with the lesion 38, steps of deflating the balloon 13 and removing the balloon catheter 10 from the patient body are performed. Then, a series of the steps is complete.

As described above, the balloon catheter 10 is generally used for blood vessel treatment, for example, treatment for coronary arteries, femoral arteries, pulmonary arteries, or other types of blood vessels; however, the balloon catheter 10 can be used for treatment of any other types of tubes in living organism other than blood vessels such as ureters and gastrointestinal tracts or body cavities.

According to the configuration of the embodiment described above in detail, the following advantageous effects can be achieved.

The outer surface of the balloon 13 includes the second regions 22 that have the greater surface roughness. With the second regions 22 that is in contact with the lesion 38, the balloon 13 is less likely to slip during the inflation of the balloon 13. Further, the second region is formed in only a portion of the balloon 13 with respect to the axial direction and any other portion of the balloon 13 includes the first region that has less surface roughness. In comparison to a configuration in which the second region 22 is formed in an entire area of the balloon 13 with respect to the axial direction, the sliding resistance of the balloon 13 during the insertion of the balloon 13 into a blood vessel is reduced. Therefore, the slipping of the balloon 13 during the inflation of the balloon 13 is reduced without a reduction in ease in passing of the balloon 13.

The straight portion 13c has the maximum diameter among portions of the balloon 13 and thus the balloon 13 is more likely to slide on a tube wall during the insertion of the balloon 13 into a blood vessel. Because the outer surface of the straight portion 13c includes the first region 21 that has less surface roughness, the sliding resistance of the balloon 13 during the insertion of the balloon 13 into the blood vessel is properly reduced. Therefore, the ease in passing of the balloon 13 is properly reduced.

The second region 22 is formed not only in the outer surface of the tip-end tapered portion 13d but also in the outer surface of the base-end tapered portion 13b. According to the configuration, even if the base-end tapered portion 13b is pushed by a lesion at an angle during dilation of a section with the lesion using the base-end tapered portion 13b by inflating the balloon 13, the base-end tapered portion 13 is less likely to slip toward the tip-end side and thus the balloon 13 is less likely to slip toward the tip-end side.

Because the second regions 22 are present for the entire area of the balloon 13 with respect to the circumferential direction of the balloon 13, the second regions 22 properly contact the lesion 38 during the inflation of the balloon 13 even if the lesion 38 is present only at a section of a circumference of a blood vessel. According to the configuration, the slipping of the balloon 13 during the inflation of the balloon 13 is properly reduced.

In the expanding process of expanding the parison 39 in the internal space 31 of the mold 30, the first region 21 is formed in the outer surface of the straight tube portion 13c and the second regions 22 are formed in the outer surfaces of the tapered portions 13b, 13d. Therefore, a downstream process such as scraping of the outer surfaces of the tapered portions 13b, 13d to form the second regions 22 is not required after the balloon 13 is formed by expanding the parison 39. Therefore, the balloon 13 that includes the first region 21 and the second regions 22 with different degrees of the surface roughness is relatively easily produced.

### Second Embodiment

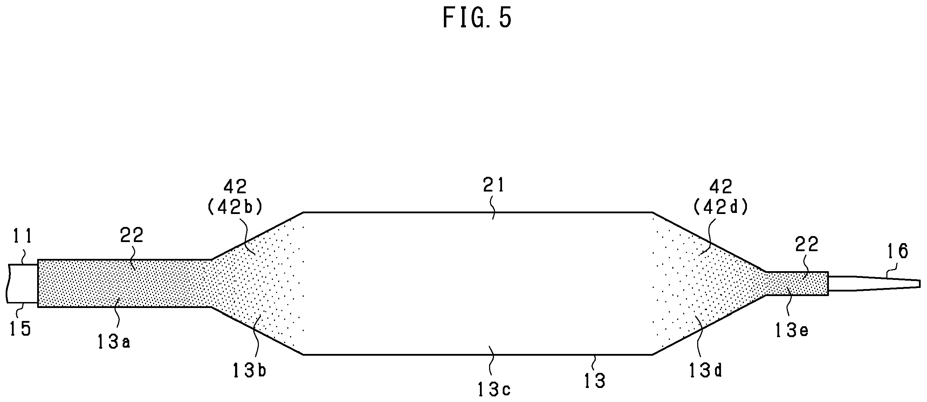

A second embodiment will be described. In this embodiment, the outer surfaces of the tapered portions 13b, 13d of the balloon 13 include second regions that have surface roughness that varies with respect to the axial direction of the balloon 13. That is a difference between the first embodiment and this embodiment, and the difference will be described below. FIG. 5 is a side view illustrating a configuration of the balloon 13 and therearound in this embodiment.

As illustrated in FIG. 5, in the balloon 13 in this embodiment, the outer surface of the straight tube portion 13c includes the first region 21 and the outer surfaces of the base-end leg portion 13a and the tip-end leg portion 13e include the second regions 22. The regions 21, 22 have configurations similar to those in the first embodiment and thus will not be described. In FIG. 5, the second regions 22 are indicated with dot-hatching patterns.

The outer surfaces of the base-end tapered portion 13b and the tip-end tapered portion 13d of the balloon 13 in this embodiment include second regions 42 with surface roughness greater than the surface roughness of the first region 21. In FIG. 5, the second regions 42 are indicated with dot-hatching patterns. The second regions 42 include a second region 42b in an entire area of the outer surface of the base-end tapered portion 13b. The surface roughness of the second region 42b of the base-end tapered portion 13b decreases from the base-end side toward the tip-end side. The surface roughness of the second region 42b is greater than the surface roughness of the first region 21 of the straight tube portion 13c for the entire area of the second region 42b. The surface roughness of the second region 42b is less than the surface roughness of the second region 22 of the base-end leg portion 13a for the entire area of the second region 42b.

The surface roughness of the second region 42d of the tip-end tapered portion 13d gradually decreases from the tip-end side toward the base-end side. The second region 42d is present for the entire area of the outer surface of the tip-end tapered portion 13d. The surface roughness of the second region 42d is greater than the surface roughness of the first region 21 of the straight tube portion 13c for the entire area of the second region 42d. The surface roughness of the second region 42d is less than the surface roughness of the second region 22 of the tip-end leg portion 13e for the entire area of the second region 42d.

As described above, the surface roughness of the second regions 42b, 42d of the tapered portions 13b, 13d gradually decreases toward the straight tube portion 13c. That is, the surface roughness of the second regions 42b, 42d of the tapered portions 13b, 13d gradually increases as a distance from the straight tube portion 13c increases.

The balloon 13 in this embodiment is produced through steps similar to the steps in the first embodiment. As described above, the surface roughness of the second regions 42b, 42d of the tapered portions 13b, 13d of the balloon 13 in this embodiment is different from the surface roughness of the balloon in the first embodiment. Therefore, the mold 30 (see FIG. 3) that includes the inner wall surfaces 37b, 37d that have the surface roughness that corresponds to the surface roughness of the second regions 42b, 42d is used for the production of the balloon 13.

According to the configuration of this embodiment as described above in detail, the following advantageous effects can be achieved.

In the balloon 13, the outer surface of the straight tube portion 13 includes the first region 21 that has the less surface roughness, the outer surfaces of the tapered portions 13b, 13d include the second regions 42 that have the greater surface roughness, and the outer surfaces of the leg portions 13a, 13e include the second regions 22 that have the greater surface roughness. According to the configuration, the effects similar to the effects achieve by the first embodiment can be achieved.

The diameter of sections of the tapered portions 13b, 13d adjacent to the straight tube portion 13c is relatively larger. Therefore, the sections adjacent to the straight tube portion 13c may be more likely to slide on a tube wall during the insertion of the balloon 13 into a blood vessel. This embodiment is conceived in view of such circumstances and the surface roughness of the second regions 42b, 42d of the tapered portions 13b, 13d decreases toward the straight tube portion 13c. According to the configuration, the sliding resistance of the sections of the tapered portions 13b, 13d adjacent to the straight tube portion 13c during the insertion of the balloon 13 into a blood vessel can be reduced. Therefore, the sliding resistance of the balloon 13 during the insertion of the balloon 13 into the blood vessel can be further reduced.

The diameter of the tip-end tapered portion 13d is smaller at the section farther from the straight tube portion 13c (i.e., the section on the tip-end side). Therefore, the section farther from the straight tube portion 13c is easily inserted into a narrowed section. In the configuration described above, the surface roughness of the second region 42d of the tip-end tapered portion 13d increases as the distance from the straight tube portion 13c increases. Therefore, during the inflation of the balloon 13 by inserting the tip-end tapered portion 13d into the narrowed section, the slipping of the tip-end tapered portion 13d toward the base-end side is properly reduced and thus the slipping of the balloon 13 toward the base-end side is properly reduced.

### Third Embodiment

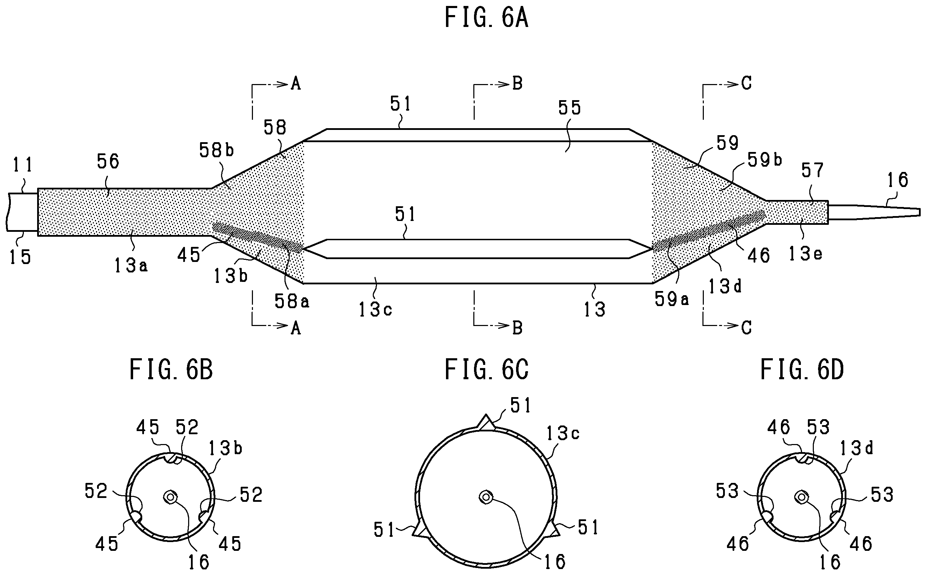

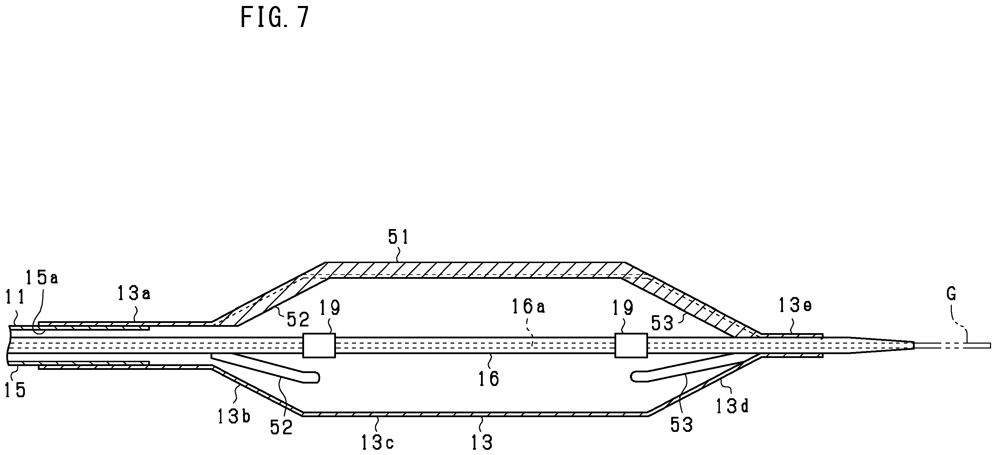

A third embodiment will be described. This embodiment includes linear protrusions on the outer surface and the inner surface of the balloon 13. First, the protrusions will be described with reference to FIGS. 6 and 7. FIG. 6(a) is a side view illustrating a configuration of the balloon 13 and therearound in this embodiment. FIG. 6(b) is a cross-sectional view cut along line A-A in FIG. 6(a). FIG. 6(c) is a cross-sectional view cut along line B-B in FIG. 6(a). FIG. 6(d) is a cross-sectional view cut along line C-C in FIG. 6(a). In FIGS. 6(a) to 6(d), the balloon 13 is inflated. FIG. 7 is a side view illustrating the balloon 13 and therearound with a lengthwise cross section of the balloon 13 and the outer tube 15.

As illustrated in FIGS. 6(a) to 6(d) and 7, the balloon 13 includes external protrusions 51 on the straight tube portion 13c, internal protrusions 52 on the base-end tapered portion 13b, and internal protrusions 53 on the tip-end tapered portion 13d. The protrusions 51 to 53 are integrally formed with the balloon 13.

The external protrusions 51 protrude from the outer surface of the straight tube portion 13c and extend along the outer surface of the straight tube portion 13c in the axial direction of the balloon 13. Multiple (specifically, three) external protrusions 51 are disposed at predetermined intervals (specifically, at equal intervals) in the circumferential direction of the balloon 13. Each external protrusion 51 has a widthwise cross section (specifically, a cross section along a direction perpendicular to the longitudinal direction of the external protrusion 51) in an inverted V-shape that protrudes outward in the radial direction of the balloon 13, specifically, in a triangular shape.

The external protrusions 51 are for creating incisions in a lesion during inflation of the balloon 13 to dilate a section with a lesion. With the balloon catheter 10, the section with the lesion can be easily dilated by creating incisions in the lesion using the external protrusions 51 and using the incisions as a trigger of the dilation. That is, the balloon catheter 10 is a balloon catheter having a scoring function.

The internal protrusions 52 on the base-end tapered portion 13b protrude from an inner surface of the base-end tapered portion 13b and extend along the inner surface of the base-end tapered portion 13b in the axial direction of the balloon 13. Specifically, the internal protrusions 52 extend for an entire length in the axial direction of the base-end tapered portion 13b. Multiple (specifically, three) internal protrusions 52 are disposed at predetermined intervals (specifically, equal intervals) in the circumferential direction of the balloon 13. The internal protrusions 52 are disposed at positions at which corresponding external protrusions 51 are located with respect to the circumferential direction of the balloon 13. Each internal protrusion 52 has a widthwise cross section (specifically, a cross section along a direction perpendicular to the longitudinal direction of the internal protrusion 52) in a semicircular shape that protrudes toward an inner side of the balloon 13.

The internal protrusion 53 on the tip-end tapered portion 13d protrude from the inner surface of the tip-end tapered portion 13d and extend along the inner surface of the tip-end tapered portion 13d in the axial direction of the balloon 13. Specifically, the internal protrusions 53 extend for an entire length in the axial direction of the tip-end tapered portion 13d. Multiple (specifically, three) internal protrusions 53 are disposed at predetermined intervals (specifically, equal intervals) in the circumferential direction of the balloon 13. The internal protrusions 53 are disposed at positions at which corresponding external protrusions 51 are located with respect to the circumferential direction of the balloon 13. Each internal protrusion 53 has a widthwise cross section (specifically, a cross section along a direction perpendicular to the longitudinal direction of the internal protrusion 53) in a semicircular shape that protrudes toward an inner side of the balloon 13.

The outer surface of the balloon 13 includes a first region 55 with less surface roughness and second regions 56 to 59 with surface roughness greater than the surface roughness of the first region 55. The regions 55 to 59 will be described with reference to FIG. 6(a). In FIG. 6(a), the second regions 56 to 59 are indicated with dot-hatching patterns.

As illustrated in FIG. 6(a), in the balloon 13, the outer surface of the straight tube portion 13c includes the first region 55, the outer surface of the base-end leg portion 13a includes the second region 56, the outer surface of the tip-end leg portion 13e includes the second region 57, the outer surface of the base-end tapered portion 13b includes the second region 58, and the outer surface of the tip-end tapered portion 13d includes the second region 59. The first region 55 of the straight tube portion 13c is present for the entire area of the outer surface of the straight tube portion 13c. The surface roughness of the first region 55 is constant for the entire area of the first region 55.

The second region 56 of the base-end leg portion 13a is present for the entire area of the outer surface of the base-end leg portion 13a. The second region 57 of the tip-end leg portion 13e is present for the entire area of the outer surface of the tip-end leg portion 13e. The second regions 56 and 57 have the same surface roughness. The surface roughness of the second regions 56 and 57 is constant for the entire area of the second regions 56 and 57.

The second region 58 of the base-end tapered portion 13b is present for the entire area of the outer surface of the base-end tapered portion 13b. Regions of the outer surface of the base-end tapered portion 13b overlapping the internal protrusions 52 with respect to the thickness direction of the base-end tapered portion 13b, respectively, are defined as overlapping regions 45. Specifically, each overlapping region 45 is located on an opposite side from a side on which the corresponding internal protrusion 52 protrudes and is overlapped with the corresponding internal protrusion 52 with respect to the thickness direction. The outer surface of the base-end tapered portion 13b includes the overlapping regions 45 that overlap the respective internal protrusions 52. Each overlapping region 45 is elongated along the corresponding internal protrusion 52.

The second region 58 of the base-end tapered portion 13b includes multiple (specifically, three) second regions 58a in the overlapping regions 45 of the outer surface of the base-end tapered portion 13b and second regions 58b in regions of the outer surface of the base-end tapered portion 13b other than the overlapping regions 45. The surface roughness of each second region 58a is greater than the surface roughness of the second regions 58b. The second regions 58a have the same surface roughness. The second regions 58a correspond to "a predetermined area in the overlapping region" and the second region 58b corresponds to "any other area."

The second region 59 of the tip-end tapered portion 13d is present for the entire outer surface of the tip-end tapered portion 13d. Sections of the outer surface of the tip-end tapered portion 13d overlap the internal protrusions 53 with respect to the thickness direction of the tip-end tapered portion 13d, respectively. The sections are defined as overlapping regions 46. Specifically, each overlapping region 46 is located on an opposite side from a side on which the corresponding internal protrusion 53 protrudes, and each overlapping region 46 is overlapped with the corresponding internal protrusion 53 with respect to the thickness direction. The outer surface of the tip-end tapered portion 13d includes the overlapping regions 46 that overlap the respective internal protrusions 53. Each overlapping region 46 is elongated along the corresponding internal protrusion 53.

The second region 59 of the tip-end tapered portion 13d includes multiple (specifically, three) second regions 59a in the overlapping regions 46 of the outer surface of the tip-end tapered portion 13d and second regions 59b in regions of the outer surface of the tip-end tapered portion 13d other than the overlapping regions 46. The surface roughness of each second region 59a is greater than the surface roughness of the second regions 59b. The second regions 59a have the same surface roughness. The second regions 59a correspond to "a predetermined area in the overlapping region" and the second region 59b corresponds to "any other area."

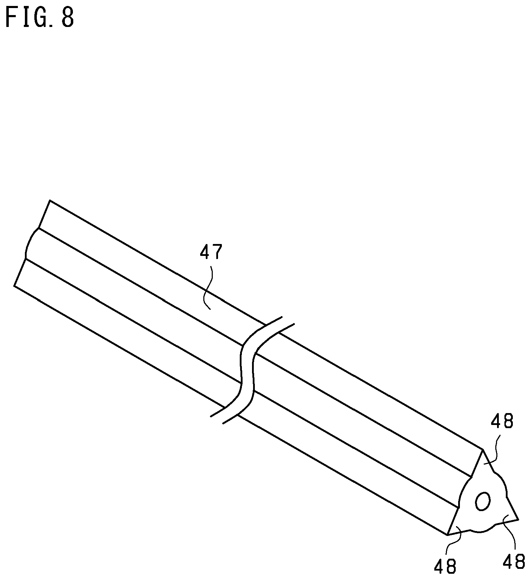

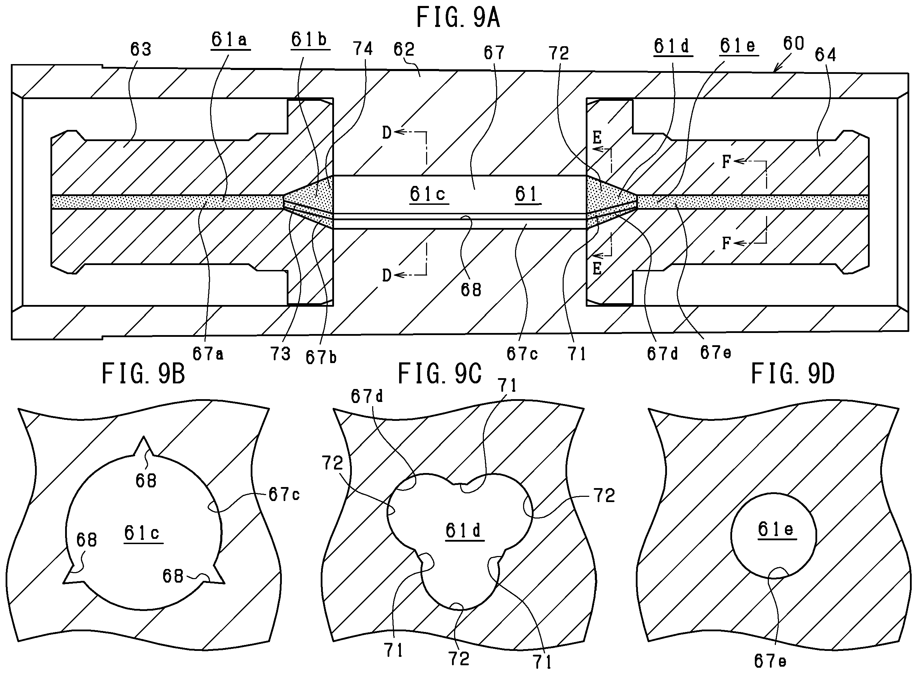

A method of producing the balloon 13 in this embodiment will be described. Because the external protrusion 51 and the internal protrusions 52, 53 are disposed on the balloon 13, configurations of a parison 47 and a mold 60 in this embodiment are different from those in the first embodiment. Therefore, the configurations of the parison 47 and the mold 60 will be described first. FIG. 8 is a perspective view of the parison 47. FIG. 9(a) is a cross-sectional view illustrating the configuration of the mold 60. FIG. 9(b) is a cross-sectional view cut along line D-D in FIG. 9(a). FIG. 9(c) is a cross-sectional view cut along line E-E in FIG. 9(a). FIG. 9(d) is a cross-sectional view cut along line F-F in FIG. 9(a).

First, the configuration of the parison 47 will be described with reference to FIG. 8. As illustrated in FIG. 8, the parison 47 is made of a resin material and formed in a substantially cylindrical shape. The parison 47 may be formed by extrusion molding. The parison 47 includes multiple (specifically, three) protrusions 48 that protrude from the outer periphery of the parison 47. Each protrusion 48 has a cross section (specifically, a cross section along a direction perpendicular to the longitudinal direction of the parison 47) in an inverted V-shape (specifically, a triangular shape), which protrudes outward in the radial direction of the parison 47. The protrusion 48 extends in the longitudinal direction of the parison 47, more specifically, extends for the entire length in the longitudinal direction of the parison 47. The protrusions 48 are to be formed into the external protrusion 51 and the internal protrusions 52, 53 of the balloon 13.

Next, the configuration of the mold 60 will be described with reference to FIGS. 9(a) to 9(d). As illustrated in FIG. 9(a), the mold 60 has the configuration basically similar to that of the mold 30 in the first embodiment. The mold 60 includes an internal space 61 for forming the balloon 13. The internal space 61 extends in the longitudinal direction of the mold 60. The internal space 61 includes a space section 61a for forming the base-end leg portion 13a, a space section 61b for forming the base-end tapered portion 13b (corresponding to a second space section), a space section 61c for forming the straight tube portion 13c (corresponding to a first space section), a space section 31d for forming the tip-end tapered portion 13d (corresponding to a second space section), and a space section 61e for forming the tip-end leg portion 13e.

The mold 60 includes multiple mold components 62 to 64 that define the internal space 61. The mold component 62 includes a space section 61c for forming the straight tube portion 13c. The mold component 63 includes a space section 61b for forming the base-end tapered portion 13b and a space section 61a for forming the base-end leg portion 13a. The mold component 64 includes a space section 61d for forming the tip-end tapered portion 13d and the space section 61e for forming the tip-end leg portion 13e.

The mold 60 includes an inner wall surface 67 that defines the internal space 61. The inner wall surface 67 includes an inner wall surface 67a that defines the space section 61a for the base-end leg portion 13a, an inner wall surface 67b that defines the space section 61b for the base-end tapered portion 13b (corresponding to a second surface section), an inner wall surface 67c that defines the space section 61c for the straight tube portion 13c (corresponding to a first surface section), an inner wall surface 67d that defines the space section 61d for the tip-end tapered portion 13d (corresponding to a second surface section), and an inner wall surface 67e that defines the space section 61e for the tip-end leg portion 13e. The surface roughness of the inner wall surface 67c is less than the surface roughness of other inner wall surfaces 67a, 67b, 67d and 67e. The surface roughness of each of the inner wall surfaces 67a, 67b, 67d and 67e is greater than the surface roughness of the inner wall surface 67c. The inner wall surfaces 67a, 67b, 67d and 67e have the same surface roughness. In FIG. 9(a), the inner wall surfaces 67a, 67b, 67d and 67e are indicated with dot-hatching patterns.

In the mold 60 in this embodiment, shapes of cross sections of the space sections 61a to 61e of the internal space 61 (or cross sections of the inner wall 67) are different from one another. Therefore, configurations of the space sections 61a to 61e will be described in sequence below.

First, the space section 61c for the straight tube portion 13c will be described with reference to FIG. 9(b). As illustrated in FIG. 9(b), the space section 61c for the straight tube portion 13c has a cross section (specifically, the cross section along a direction perpendicular to the longitudinal direction of the internal space 61) in a circular shape. Multiple (specifically, three) grooves 68 are formed in the inner wall surface 67c that defines the space section 61c. The grooves 68 extend in the longitudinal direction of the internal space 61, specifically, for an entire length of the internal wall surface 67c in the longitudinal direction. The grooves 68 are at equal intervals in the circumferential direction of the inner wall surface 67c. Each groove 68 has a cross section in a triangular shape that protrudes outwards on an outer periphery side of the space section 61c. The grooves 68 are formed only in the inner wall surface 67c and not in other inner wall surfaces 67a, 67b, 67d, and 67e.

Next, the space section 61d for the tip-end tapered portion 13d will be described with reference to FIGS. 9(a) and 9(c). As illustrated in FIG. 9(a), the inner wall surface 67d that defines the space section 61d for the tip-end tapered portion 13d includes a slope surface that is angled relative to the axial direction of the internal space 61. The slope surface (a tapered surface) is at an angle corresponding to an angle of the outer surface of the tip-end tapered portion 13d.

As illustrated in FIG. 9(c), the space section 61d has a cross section in a non-circular shape. The inner wall surface 67d that defines the inner space section 61d includes protruded surfaces 71 that are parts of the inner wall surface 61d with respect to the circumferential direction of the inner wall surface 61d protruded inward. The protruded surfaces 71 extend in the axial direction of the internal space 61 at an angle along the angle of the inner wall surface 67d. The protruded surfaces 71 include flat surfaces. Specifically, the protruded surfaces 71 extend for an entire length of the inner wall surface 67d in the axial direction of the inner wall surface 67d. Multiple (specifically, three) protruded surfaces 71 are at equal intervals in the circumferential direction of the inner wall surface 67d. The protruded surfaces 71 are at positions at which the grooves 68 are located with respective to the circumferential direction of the inner wall surface 67d (i.e., the circumferential direction of the internal space 61).

Surfaces of the inner wall surface 67d connecting one protruded surface 71 to another are concaved surfaces 72 each having arc-shaped cross sections that are concaved outward in the inner wall surface 67d. Multiple (specifically, three) concaved surfaces 72 are present and each concave surface 72 is located between adjacent two of the protruded surfaces 71.

The surface roughness of the inner wall surface 67d is greater than the surface roughness of the inner wall surface 67c. The surface roughness of the inner wall surface 67d is constant for an entire area of the inner surface section 67d that includes the protruded surfaces 71 and the concaved surfaces 72.

Next, the space section 61b for the base-end tapered portion 13b will be described. As illustrated in FIG. 9(a), the inner wall surface 67b that defines the space section 61b for the base-end tapered portion 13b includes a sloped surface that is angled relative to the axial direction of the internal space 61. The sloped surface (a tapered surface) is at an angle corresponding to an angle of the outer surface of the base-end tapered portion 13b.

Although not illustrated, the space section 61b has a cross section in a shape similar to the shape of the cross section of the space section 61d for the tip-end tapered portion 13d. The inner wall surface 67b that defines the space section 61b includes protruded surfaces 73 that protrude inward. The protruded surfaces 73 extend in the axial direction of the internal space 61 along the slope of the inner wall surface 67b and include flat surfaces. Specifically, the protruded surfaces 73 extend for an entire length of the inner wall surface 67b in the axial direction of the inner wall surface 67b. Multiple (specifically, three) protruded surfaces 73 are at equal intervals in the circumferential direction of the inner wall surface 67b. The protruded surfaces 73 are at positions at which the grooves 68 are located with respect to the circumferential direction of the inner wall surface 67b.

Surfaces of the inner wall surface 67b connecting one protruded surface 73 to another are concaved surfaces 74 each having arc-shaped cross sections that are concaved outward in the inner wall surface 67b. Multiple (specifically, three) concaved surfaces 74 are present and each concave surface 74 is located between adjacent two of the protruded surfaces 73.

The surface roughness of the inner wall surface 67b is greater than the surface roughness of the inner wall surface 67c. The surface roughness of the inner wall surface 67b is constant for an entire surface of the inner surface section 67b that includes the protruded surfaces 73 and the concaved surfaces 74.

As illustrated in FIG. 9(d), the space section 61e for the tip-end leg portion 13e has a cross section in a circular shape. Similarly, the space section 61a for the base-end leg portion 13a has a cross section in a circular shape.

Next, a method of producing the balloon 13 using the mold 60 in this embodiment will be described.

First, a preparing process is performed to prepare the mold 60 and the parison 47. Then, a placing process is performed to place the parison 47 in the internal space 61 of the mold 60. In this process, the parison 47 is placed in the internal space 61 with the longitudinal direction of the parison 47 aligned with the longitudinal direction of the internal space 61. Therefore, the parison 47 is placed over the space sections 61a to 61e in the internal space 61.

Next, an expanding process is performed to heat and expand the parison 47 in the internal space 61 of the mold 60. Through this process, an expanded parison that includes featured portions of the balloon 13 is formed, that is, the expanded parison that includes the portions 13a to 13e of the balloon 13 is formed.

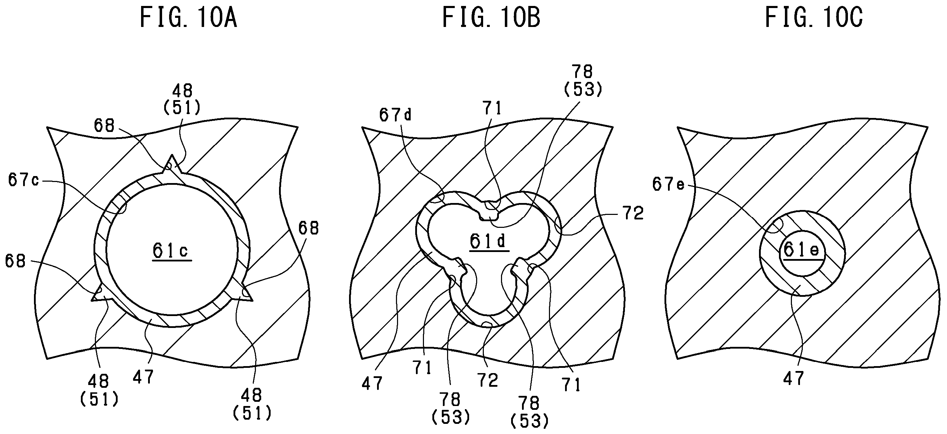

Steps of the expanding process will be described in detail with reference to FIGS. 10(a) to 10(c). FIGS. 10(a) to 10(c) are cross-sectional views illustrating the parison 47 that is expanded in the internal space 61 of the mold 60. FIG. 10(a) is the cross-sectional view cut along line D-D in FIG. 9(a). FIG. 10(b) is the cross-sectional view cut along line E-E in FIG. 9(a). FIG. 10(c) is the cross-sectional view cut along line F-F in FIG. 9(a).

In the expanding process, the outer periphery of the parison 47 that is expanded is pressed against the inner wall surface 67c of the mold 60 (the mold component 62) to form the first region 55 in the outer surface of the straight tube portion 13c. As illustrated in FIG. 10(a), the protrusions 48 of the parison 47 are inserted into the grooves 68, respectively, to form the external protrusions 51. That is, the protrusions 48 inserted into the respective grooves 68 are formed into the external protrusions 51.

In the expanding process, the outer periphery of the parison 47 that is expanded is pressed against the inner wall surface 67d of the mold 60 (the mold component 64) to form the second region 59 in the outer surface of the tip-end tapered portion 13d. As illustrated in FIG. 10(b), the protrusions 48 of the parison 47 are pressed against the protruded surfaces 71 of the mold 60, respectively, until the protrusions 48 are squashed and portions of the parison 47 along the protrusions 48 protrude inward to form the internal protrusions 53. During the squashing of the protrusions 48, the second regions 59a are formed in surfaces that are in close contact with the protruded surfaces 71. Through the steps, the second regions 59a are formed in the overlapping regions 46 of the tip-end tapered portion 13d.

In the expanding process, the outer periphery of the parison 47 that is expanded is pressed against the inner wall section 67b of the mold 60 (the mold component 63) to form the second region 58 in the outer surface of the base-end tapered portion 13b. Specifically, the protrusions 48 of the parison 47 are pressed against the protruded surfaces 73 of the mold 60, respectively, until the protrusions 48 are squashed and portions of the parison 47 along the protrusions 48 protrude inward to form the internal protrusions 52. During the squashing of the protrusions 48, the second regions 58a are formed in surfaces that are in close contact with the protruded surfaces 73. Through the steps, the second regions 58a are formed in the overlapping regions 45 of the base-end tapered portion 13b.

In the expanding process, the outer periphery of the parison 47 that is expanded is pressed against the inner wall section 67e of the mold 60 (the mold component 64) to form the second region 57 in the outer surface of the tip-end leg portion 13e. The outer periphery of the parison 47 that is expanded is pressed against the inner wall section 67a of the mold 60 (the mold component 63) to form the second region 56 in the outer surface of the base-end leg portion 13a. During the above steps, the protrusions 48 of the parison 47 are pressed against the inner wall sections 67a, 67e and squashed.

After the expanding process, a cutting step is performed to cut off extra end portions or the expanded parison. This completes the formation of the balloon 13 and the production of the balloon 13 is complete.

Downstream processes including a joining process for joining the balloon 13 to the catheter tube 11 and a joining process for joining the catheter tube to the hub 12 are performed. At the completion of the downstream processes, a series of production steps is complete.

According to the configuration of the embodiment described in detail above, the following advantageous effects can be achieved.

In the balloon 13, the first region 55 that has the less surface roughness is formed in the outer surface of the straight tube portion 13 and the second regions 55 to 59 that have the greater surface roughness are formed in the outer surfaces of the tapered portions 13b, 13d and the leg portions 13a, 13e, respectively. According to such a configuration, effects similar to the effects achieved by the first embodiment can be achieved.

In the balloon 13, the internal protrusions 52, 53 protrude from the inner surfaces of the tapered portions 13b, 13d and extend in the axial direction. The sections of the tapered portions 13b, 13d at which the internal protrusions 52, 53 are disposed have greater strength. Therefore, the sections of the tapered portions 13b, 13d are properly contact the lesion 38 during the dilation of the section with the lesion 38 using the tapered portions 13b, 13d of the balloon 13 that is inflated. According to the configuration, the tapered portions 13b, 13d are less likely to be pushed back by the lesion 38 during the dilation of the section with the lesion 38 using the tapered portions 13b, 13d.

During the dilation of the section with the lesion 38 using the tapered portions 13b, 13d, the overlapping regions 45, 46, in which the internal protrusions 52, 53 and the outer surfaces of the tapered portions 13b, 13d overlap in the thickness direction of the tapered portions 13b, 13d, properly contact the lesion 38. In this embodiment, the second regions 58, 59 are formed in areas of the outer surfaces of the tapered portions 13b, 13d that includes the overlapping regions 45, 46 (specifically, the entire areas of the outer surfaces of tapered portions 13b, 13d). According to the configuration, the second regions 58, 59 in the overlapping regions 45, 46 are properly contact the lesion 38. Therefore, the slipping of the balloon 13 is properly reduced.

Among the second regions 58, 59 of the tapered portions 13b, 13d, the second regions 58a, 59a formed in the overlapping regions 45, 46 have the surface roughness greater than the surface roughness of other regions 58b, 59b. Because the surface roughness in the overlapping regions 45, 46 that properly contact the lesion 38 is greater, the advantageous effect in reduction of the slipping of the balloon 13 is enhanced.

The internal protrusions 52, 53 on the tapered portions 13b, 13d and the external protrusions 51 on the straight tube portion 13c are disposed at the same positions with respect to the circumferential direction of the balloon 13. Because the internal protrusions 52, 53 that have greater strength and the external protrusions 51 are disposed in a row with respect to the axial direction, the tapered portions 13b, 13d are further less likely to be pushed back by the lesion 38 during the dilation of the section with the lesion 38 using the tapered portions 13b, 13d.

### Other Embodiments

The present disclosure is not limited to the above embodiments and may be implemented as the followings, for example.

The second regions that have the greater surface roughness are formed in the outer surfaces of the tapered portions 13b, 13d in each of the above embodiments; however, the second regions may be formed in the outer surface of one of the tapered portions 13b, 13d and the first region that has the less surface roughness may be formed in the outer surface of the other one of the tapered portions 13b, 13d.

The second regions are formed in the outer surfaces of the leg portions 13a, 13e in each of the above embodiments; however, the first region may be formed in the outer surface of any one of or both of the leg portions 13a, 13e.

The surface roughness of the second regions 42b, 42d of the tapered portions 13b, 13d decreases toward the straight tube portion 13c in the second embodiment; however, such a configuration may be altered. For example, the first region may be formed in an area of each of the outer surfaces of the tapered portions 13b, 13d adjacent to the straight tube portion 13c and the second region may be formed in an area of each of the outer surfaces of the tapered portions 13b, 13d farther from the straight tube portion 13c. According to such a configuration, the effects similar to the effects achieve by the second embodiment can be achieved.

The first region is present for the entire area of the outer surface of the straight tube portion 13c in each of the above embodiments; however, the first region may be present in only a part of the entire area of the outer surface. For example, the first region may be present in an area of the outer surface of the straight tube portion 13c in the middle with respect to the axial direction and the second regions may be formed in parts of the entire area on the base-end side and the tip-end side with respect to the axial direction. According to such a configuration, the advantageous effect in reduction of the slipping of the balloon 13 is enhanced.

The second regions are present for the entire areas of the outer surfaces of the tapered portions 13b, 13d with respect to the circumferential direction of the balloon 13; however, such a configuration may be altered, that is, the second regions may be formed in parts of the entire areas of the outer surfaces of the tapered portions 13b, 13d with respect to the circumferential direction of the balloon 13, and the first region may be present in each of other areas of the outer surfaces of the tapered portions 13b, 13d.

The internal protrusions 52, 53 are disposed on the tapered portions 13b, 13d in the third embodiment; however, the internal protrusions may be disposed only on one of the tapered portions 13b, 13d.

The inner wall section 67 of the mold 60 includes the protruded surfaces 71 in the third embodiment; however, the protruded surfaces 71 may not be included. For example, the inner wall section 67d (or the space section 61d) may have a cross section in a circular shape. The internal protrusions 53 may be formed by pressing the protrusions 48 of the parison 47 against the inner wall section 67d and squashed during expansion of the parison 47. Alternatively, the second regions 59a may be formed in surfaces that are in close contact with the inner wall section 67d during pressing and squashing of the protrusions 48.

Similarly, the inner wall section 67b of the mold 60 may not include the protruded surfaces 73 and thus may have a cross section in a circular shape.

The first region and the second regions are formed in the surfaces of the balloon 13 using the mold in the expanding process in each of the above embodiments; however, a method of forming the regions is not limited to the method described above. For example, the first region and the second regions may be formed by scraping the outer surfaces of the balloon 13 after the production of the balloon 13 is complete.

The present disclosure has been described with reference to the embodiments. However, the present disclosure is not limited to the embodiments or the structures. Various modifications may be within in the technical scope of the present disclosure, as long as they fall under the scope of the appended claims . Further, various combinations, configurations, and other combinations and configurations including single elements, more or less, may be within the technical scope of the present disclosure.

### Reference Sings List

10: Balloon catheter, 11: Catheter tube, 13: Balloon, 13a: Base-end leg portion, 13b: Base-end tapered portion, 13c: Straight tube portion, 13d: Tip-end tapered portion, 13e: Tip-end leg portion, 21: First region, 22: Second region, 30: Mold, 31: Internal space, 42b: Second region, 42d: Second region, 51: External protrusion, 52: Internal protrusion, 53: Internal protrusion, 55: First region, 56-59: Second region, 60: Mold, 61: Internal space

## Claims

1. A balloon catheter comprising a balloon (13) that is inflatable and deflatable, wherein
the balloon (13) includes:
a straight tube portion (13c) that has a diameter that is to be maximum when the balloon is inflated; and
two tapered portions (13b, 13d) that sandwich the straight tube portion (13c) with respect to an axial direction, wherein the tapered portions (13b, 13d) decrease in diameter toward farther sides from the straight tube portion (13c), wherein
the balloon (13) includes an outer surface that includes a first region (21) and a second region (22, 42) that is at a part of the balloon (13) in the axial direction of the balloon (13) and has surface roughness greater than surface roughness of the first region, wherein
the second region (22, 42) is formed in an outer surface of one of the tapered portions (13b, 13d),
wherein the first region (21) is formed in an outer surface of the straight tube portion (13c), and
wherein the second region (22, 42) of one of the tapered portions (13b, 13d) has surface roughness that decreases toward the straight tube portion (13c).

2. The balloon catheter according to claim 1, wherein the second region (22, 42) is formed in the outer surface of the one of the tapered portions (13d) on the tip-end side.

3. The balloon catheter according to claim 2, further comprising a catheter tube (11) that includes a tip-end portion at which the balloon (13) is disposed, wherein
the balloon (13) includes a tip-end leg portion (13e) that is on an opposite side of the one of the tapered portions (13d) on the tip-end side from the straight tube portion (13c), wherein the tip-end leg portion (13e) is joined to the catheter tube (11), and
the second region (22, 42) is formed in an outer surface of the tip-end leg portion (13e).

4. The balloon catheter according to claim 1, further comprising a catheter tube (11) that includes a tip-end portion at which the balloon (13) is disposed, wherein
the balloon (13) includes a leg portion (13a) that is on an opposite side of one of the tapered portions (13b, 13d) from the straight tube portion (13c), wherein the leg portion (13a) is joined to the catheter tube (11), and
the second region (22, 42) is formed in an outer surface of the leg portion (13a).

5. A method of producing the balloon catheter according to one of claims 1 to 3, the method comprising producing the balloon (13) using a mold (30) that includes an internal space (31) for forming the balloon (13), wherein
the internal space (31) includes a first space section (31c) for forming the straight tube portion (13c) and a second space section (31b, 31d) for forming one of the tapered portions (13b, 13d),
the mold (30) includes an inner wall surface (37) that defines the internal space (31),
the inner wall surface (37) includes a first surface section (37c) that defines the first space section (31c) and a second surface section (37b, 37d) that defines the second space section (31b, 31d),
the second surface section (37b, 37d) has surface roughness greater than surface roughness of the first surface section (37c), wherein the method comprises:
a placing process of placing a parison (39) in the internal space (31), wherein the parison (39) in a tubular shape is a base material of the balloon (13);
an expanding process of expanding the parison (39) in the internal space (31), wherein the expanding process includes forming the first region (21) in the outer surface of the straight tube portion (13c) by pressing an outer periphery of the parison (39) against the first surface section (37c), wherein the parison is expanded, and forming the second region (22, 42) in the outer surface of one of the tapered portions (13b, 13d) by pressing the outer periphery of the parison (39) against the second wall section, wherein the parison (39) is expanded.

6. The method according to claim 5, wherein the method is for producing the balloon catheter that includes an external protrusion (51) that protrudes from the outer surface of the straight tube portion (13c) and extends in the axial direction and an internal protrusion (52, 53) that protrudes from an inner surface of the one of the tapered portions (13b, 13d) and extends in the axial direction along the inner surface, wherein
the parison (47) includes a protrusion (48) that protrudes from an outer periphery of the parison (47) and extends in a longitudinal direction of the parison,
the first surface section includes a groove (68) that extends in a longitudinal direction of the internal space,
the second surface section does not include the groove, and
the expanding process comprises:
forming the external protrusion (51) by inserting the protrusion (48) of the parison (47) into the groove (68), wherein the parison is expanded;
forming the internal protrusion (52, 53) by pressing the protrusion (48) of the parison (47) against the second surface section until the protrusion is squashed and a portion of the parison along the protrusion protrudes inward, wherein the parison is expanded; and
forming the second region (22, 42) in a surface that is in close contact with the second surface section during squashing of the protrusion.

7. The method according to claim 6, wherein
the second surface section that defines the second space section includes a protruded surface (71, 73) that is a part of the second surface section with respect to a circumferential direction of the second surface section that is protruded inward, and
the expanding process includes pressing the protrusion (48) against the protruded surface (71, 73) until the protrusion is squashed.

## Patentansprüche

1. Ballonkatheter, der einen aufpumpbaren und entleerbaren Ballon (13) umfasst, wobei
der Ballon (13) umfasst:
einen geraden Rohrabschnitt (13c), dessen Durchmesser im aufgepumpten Zustand des Ballons seinen Maximalwert erreicht; und
zwei sich verjüngende Abschnitte (13b, 13d), die den geraden Rohrabschnitt (13c) in axialer Richtung einrahmen, wobei sich der Durchmesser der sich verjüngenden Abschnitte (13b, 13d) zu den vom geraden Rohrabschnitt (13c) weiter entfernten Seiten hin verjüngt, wobei
der Ballon (13) eine Außenfläche aufweist, die einen ersten Bereich (21) und einen zweiten Bereich (22, 42) aufweist, der sich an einem Teil des Ballons (13) in axialer Richtung des Ballons (13) befindet und eine Oberflächenrauheit aufweist, die größer ist als die Oberflächenrauheit des ersten Bereichs, wobei
der zweite Bereich (22, 42) in einer Außenfläche eines der sich verjüngenden Abschnitte (13b, 13d) ausgebildet ist,
wobei der erste Bereich (21) in einer Außenfläche des geraden Rohrabschnitts (13c) ausgebildet ist, und
wobei der zweite Bereich (22, 42) eines der sich verjüngenden Abschnitte (13b, 13d) eine Oberflächenrauheit aufweist, die zum geraden Rohrabschnitt (13c) hin abnimmt.

2. Ballonkatheter gemäß Anspruch 1, wobei der zweite Bereich (22, 42) in der Außenfläche des einen der sich verjüngenden Abschnitte (13d) auf der Spitzenendseite ausgebildet ist.

3. Ballonkatheter gemäß Anspruch 2, weiter umfassend einen Katheterschlauch (11), der einen Spitzenendabschnitt aufweist, an dem der Ballon (13) angeordnet ist, wobei
der Ballon (13) einen Spitzenend-Schenkelabschnitt (13e) aufweist, der sich auf einer dem geraden Rohrabschnitt (13c) gegenüberliegenden Seite des einen der sich verjüngenden Abschnitte (13d) auf der Spitzenendseite befindet, wobei der Spitzenend-Schenkelabschnitt (13e) mit dem Katheterschlauch (11) verbunden ist, und
der zweite Bereich (22, 42) in einer Außenfläche des Spitzenend-Schenkelabschnitts (13e) ausgebildet ist.

4. Ballonkatheter gemäß Anspruch 1, weiter umfassend einen Katheterschlauch (11), der einen Spitzenendabschnitt aufweist, an dem der Ballon (13) angeordnet ist, wobei
der Ballon (13) einen Schenkelabschnitt (13a) aufweist, der sich auf einer dem geraden Rohrabschnitt (13c) gegenüberliegenden Seite eines der sich verjüngenden Abschnitte (13b, 13d) befindet, wobei der Schenkelabschnitt (13a) mit dem Katheterschlauch (11) verbunden ist, und
der zweite Bereich (22, 42) in einer Außenfläche des Schenkelabschnitts (13a) ausgebildet ist.

5. Verfahren zur Herstellung des Ballonkatheters gemäß einem der Ansprüche 1 bis 3, wobei das Verfahren die Herstellung des Ballons (13) unter Verwendung einer Form (30) umfasst, die einen Innenraum (31) zum Bilden des Ballons (13) aufweist, wobei
der Innenraum (31) einen ersten Raumabschnitt (31c) zum Bilden des geraden Rohrabschnitts (13c) und einen zweiten Raumabschnitt (31b, 31d) zum Bilden eines der sich verjüngenden Abschnitte (13b, 13d) umfasst,
die Form (30) eine Innenwandfläche (37) aufweist, die den Innenraum (31) definiert,
die Innenwandfläche (37) einen ersten Oberflächenabschnitt (37c), der den ersten Raumabschnitt (31c) definiert, und einen zweiten Oberflächenabschnitt (37b, 37d), der den zweiten Raumabschnitt (31b, 31d) definiert, aufweist,
der zweite Oberflächenabschnitt (37b, 37d) eine Oberflächenrauheit aufweist, die größer ist als die Oberflächenrauheit des ersten Oberflächenabschnitts (37c), wobei das Verfahren umfasst:
einen Platzierungsvorgang des Platzierens eines Vorformlings (39) in dem Innenraum (31), wobei der rohrförmige Vorformling (39) ein Ausgangsmaterial des Ballons (13) ist;
einen Aufweitungsvorgang zum Aufweiten des Vorformlings (39) im Innenraum (31), wobei der Aufweitungsvorgang das Bilden des ersten Bereichs (21) in der Außenfläche des geraden Rohrabschnitts (13c) durch Drücken eines Außenumfangs des Vorformlings (39) gegen den ersten Oberflächenabschnitt (37c) umfasst, wobei der Vorformling aufgeweitet wird, und Bilden des zweiten Bereichs (22, 42) in der Außenfläche eines der sich verjüngenden Abschnitte (13b, 13d) durch Drücken des Außenumfangs des Vorformlings (39) gegen den zweiten Wandabschnitt, wobei der Vorformling (39) aufgeweitet wird.

6. Verfahren gemäß Anspruch 5, wobei das Verfahren zur Herstellung eines Ballonkatheters dient, der einen äußeren Vorsprung (51) umfasst, der von der Außenfläche des geraden Rohrabschnitts (13c) vorsteht und sich in der axialen Richtung erstreckt, und einen inneren Vorsprung (52, 53), der von einer Innenfläche des einen der sich verjüngenden Abschnitte (13b, 13d) vorsteht und sich in der axialen Richtung entlang der Innenfläche erstreckt, wobei
der Vorformling (47) einen Vorsprung (48) aufweist, der von einem Außenumfang des Vorformlings (47) vorsteht und sich in einer Längsrichtung des Vorformlings erstreckt,
der erste Oberflächenabschnitt eine Nut (68) aufweist, die sich in einer Längsrichtung des Innenraums erstreckt,
der zweite Oberflächenabschnitt die Nut nicht aufweist, und
der Aufweitungsvorgang umfasst:
Bilden des äußeren Vorsprungs (51) durch Einführen des Vorsprungs (48) des Vorformlings (47) in die Nut (68), wobei der Vorformling aufgeweitet wird;
Bilden des inneren Vorsprungs (52, 53) durch Drücken des Vorsprungs (48) des Vorformlings (47) gegen den zweiten Oberflächenabschnitt, bis der Vorsprung zusammengedrückt wird und ein Abschnitt des Vorformlings entlang des Vorsprungs nach innen vorsteht, wobei der Vorformling aufgeweitet wird; und
Bilden des zweiten Bereichs (22, 42) in einer Oberfläche, die während des Zusammendrückens des Vorsprungs in engem Kontakt mit dem zweiten Oberflächenabschnitt ist.

7. Verfahren gemäß Anspruch 6, wobei
der zweite Oberflächenabschnitt, der den zweiten Raumabschnitt definiert, eine vorstehende Oberfläche (71, 73) umfasst, die in Bezug auf eine Umfangsrichtung des zweiten Oberflächenabschnitts ein nach innen vorstehender Teil des zweiten Oberflächenabschnitts ist, und
der Aufweitungsvorgang das Drücken des Vorsprungs (48) gegen die vorstehende Oberfläche (71, 73) umfasst, bis der Vorsprung zusammengedrückt ist.

## Revendications

1. Cathéter à ballonnet comprenant un ballonnet (13) qui est gonflable et dégonflable, dans lequel
le ballonnet (13) comprend :
une partie de tube droite (13c) qui a un diamètre qui doit être maximal lorsque le ballonnet est gonflé ; et
deux parties effilées (13b, 13d) qui prennent en sandwich la partie de tube droite (13c) par rapport à une direction axiale, dans lequel les parties effilées (13b, 13d) diminuent en diamètre vers des côtés plus éloignés de la partie de tube droite (13c), dans lequel
le ballonnet (13) comprend une surface externe qui comprend une première région (21) et une seconde région (22, 42) qui est au niveau d'une partie du ballonnet (13) dans la direction axiale du ballonnet (13) et a une rugosité de surface supérieure à la rugosité de surface de la première région, dans lequel
la seconde région (22, 42) est formée dans une surface externe de l'une des parties effilées (13b, 13d),
dans lequel la première région (21) est formée dans une surface externe de la partie de tube droite (13c), et
dans lequel la seconde région (22, 42) de l'une des parties effilées (13b, 13d) a une rugosité de surface qui diminue vers la partie de tube droite (13c).

2. Cathéter à ballonnet selon la revendication 1, dans lequel la seconde région (22, 42) est formée dans la surface externe de l'une des parties effilées (13d) sur le côté d'extrémité de pointe.

3. Cathéter à ballonnet selon la revendication 2, comprenant en outre un tube de cathéter (11) qui comprend une partie d'extrémité de pointe au niveau de laquelle le ballonnet (13) est disposé, dans lequel
le ballonnet (13) comprend une partie de jambe d'extrémité de pointe (13e) qui est sur un côté opposé de l'une des parties effilées (13d) sur le côté d'extrémité de pointe par rapport à la partie de tube droite (13c), dans lequel la partie de jambe d'extrémité de pointe (13e) est jointe au tube de cathéter (11), et
la seconde région (22, 42) est formée dans une surface externe de la partie de jambe d'extrémité de pointe (13e).

4. Cathéter à ballonnet selon la revendication 1, comprenant en outre un tube de cathéter (11) qui comprend une partie d'extrémité de pointe au niveau de laquelle le ballonnet (13) est disposé, dans lequel
le ballonnet (13) comprend une partie de jambe (13a) qui est sur un côté opposé de l'une des parties effilées (13b, 13d) par rapport à la partie de tube droite (13c), dans lequel la partie de jambe (13a) est jointe au tube de cathéter (11), et
la seconde région (22, 42) est formée dans une surface externe de la partie de jambe (13a).

5. Procédé de production du cathéter à ballonnet selon l'une des revendications 1 à 3, le procédé comprenant la production du ballonnet (13) en utilisant un moule (30) qui comprend un espace interne (31) pour former le ballonnet (13), dans lequel
l'espace interne (31) comprend une première section d'espace (31c) pour former la partie de tube droite (13c) et une seconde section d'espace (31b, 31d) pour former l'une des parties effilées (13b, 13d),
le moule (30) comprend une surface de paroi interne (37) qui définit l'espace interne (31),
la surface de paroi interne (37) comprend une première section de surface (37c) qui définit la première section d'espace (31c) et une seconde section de surface (37b, 37d) qui définit la seconde section d'espace (31b, 31d),
la seconde section de surface (37b, 37d) a une rugosité de surface supérieure à la rugosité de surface de la première section de surface (37c), dans lequel le procédé comprend :
un processus de placement consistant à placer une paraison (39) dans l'espace interne (31), dans lequel la paraison (39) dans une forme tubulaire est un matériau de base du ballonnet (13) ;
un processus d'expansion consistant à expanser la paraison (39) dans l'espace interne (31), dans lequel le processus d'expansion comprend la formation de la première région (21) dans la surface externe de la partie de tube droite (13c) en pressant une périphérie externe de la paraison (39) contre la première section de surface (37c), dans lequel la paraison est expansée, et la formation de la seconde région (22, 42) dans la surface externe de l'une des parties effilées (13b, 13d) en pressant la périphérie externe de la paraison (39) contre la seconde section de paroi, dans lequel la paraison (39) est expansée.

6. Procédé selon la revendication 5, dans lequel le procédé est destiné à produire le cathéter à ballonnet qui comprend une saillie externe (51) qui fait saillie de la surface externe de la partie de tube droite (13c) et s'étend dans la direction axiale et une saillie interne (52, 53) qui fait saillie d'une surface interne de l'une des parties effilées (13b, 13d) et s'étend dans la direction axiale le long de la surface interne, dans lequel
la paraison (47) comprend une saillie (48) qui fait saillie d'une périphérie externe de la paraison (47) et s'étend dans une direction longitudinale de la paraison,
la première section de surface comprend une rainure (68) qui s'étend dans une direction longitudinale de l'espace interne,
la seconde section de surface ne comprend pas la rainure, et
le processus d'expansion comprend :
la formation de la saillie externe (51) en insérant la saillie (48) de la paraison (47) dans la rainure (68), dans lequel la paraison est expansée ;
la formation de la saillie interne (52, 53) en pressant la saillie (48) de la paraison (47) contre la seconde section de surface jusqu'à ce que la saillie soit écrasée et qu'une partie de la paraison le long de la saillie fasse saillie vers l'intérieur, dans lequel la paraison est expansée ; et
la formation de la seconde région (22, 42) dans une surface qui est en contact étroit avec la seconde section de surface pendant l'écrasement de la saillie.

7. Procédé selon la revendication 6, dans lequel
la seconde section de surface qui définit la seconde section d'espace comprend une surface en saillie (71, 73) qui est une partie de la seconde section de surface par rapport à une direction circonférentielle de la seconde section de surface qui fait saillie vers l'intérieur, et
le processus d'expansion comprend la pression de la saillie (48) contre la surface en saillie (71, 73) jusqu'à ce que la saillie soit écrasée.
